# EUROPÄISCHE PATENTSCHRIFT

(11) **EP 1 267 697 B1**
(45) Veröffentlichungstag und Bekanntmachung des Hinweises auf die Patenterteilung: **13.08.2008**
(21) Anmeldenummer: 01919411.7
(22) Anmeldetag: 30.03.2001
(51) Int. Cl.: A47L 13/17, D21H 21/24

(54) **FEUCHTTÜCHER (II)**
MOIST WIPES (II)
CHIFFONS HUMIDES (II)

(30) Priorität: 07.04.2000 DE 10017189
(43) Veröffentlichungstag der Anmeldung: 02.01.2003
(73) Patentinhaber: Cognis IP Management GmbH, 40589 Düsseldorf (DE)
(72) Erfinder: ELSNER, Michael, 42579 Heiligenhaus (DE); HANKE, Anja, 47269 Duisburg (DE); WEUTHEN, Manfred, 40764 Langenfeld (DE)
(86) Internationale Anmeldenummer: PCT/EP2001/003631
(87) Internationale Veröffentlichungsnummer: WO 2001/076445

(56) Entgegenhaltungen:
- WO-A-95/35411
- WO-A-97/30217
- US-A- 3 896 807
- US-A- 3 956 155
- US-A- 4 189 395
- US-A- 4 666 621
- US-A- 5 094 770
- US-A- 5 817 585

## Beschreibung

### Gebiet der Erfindung

Die Erfindung befindet sich auf dem Gebiet der Reinigungsmittel für harte Oberflächen und betrifft Feuchttücher, die mit einer speziellen Spezies eines nichtionischen Tensids imprägniert sind.

### Stand der Technik

Für die Reinigung von harten Oberflächen werden in der Regel mehr oder weniger viskose Flüssigkeiten eingesetzt, die direkt aufgetragen werden, von der zu reinigenden Fläche ablaufen und dabei die Hauptmenge des Schmutzes mittragen. Eine andere Anwendungsform, die sich zunehmender Bedeutung erfreut, sind Feuchttücher, sogenannte "wet wipes", bei denen es sich um textile Gewebe oder aber auch Tissuepapiere handelt, welche mit einer Reinigungsflüssigkeit getränkt sind. So werden beispielsweise in der internationalen Patentanmeldung WO 95/35411 (Procter & Gamble) Feuchttücher - wenn auch vorwiegend für kosmetische Anwendungen - vorgeschlagen, welche neben Mineralöl, Fettsäureester, Fettalkoholethoxylate und Fettalkohole enthalten.

Nachteilig bei der Anwendung dieser wet wipes ist indes, dass die eingesetzten Tenside in Form von Schlieren einen Rückstand hinterlassen, der die behandelte Fläche weniger glänzend oder sogar verschmutzt erscheinen läßt. Ein weiteres Problem tritt bei der Herstellung der Feuchttücher auf. Um das Gewebe oder Tissuepapier mit der Reinigungslösung zu tränken, wird es entweder mit ihr besprüht oder in sie eingetaucht, wobei es in beiden Fällen durch Schaumbildung oder eine zu geringe Benetzung zu einer Verminderung des Durchsatzes in der Produktion kommen kann. Eine erste Aufgabe der vorliegenden Erfindung hat somit darin bestanden, Feuchttücher unter Verwendung spezieller Tenside zur Verfügung zu stellen, welche frei sind von den eingangs geschilderten Problemen.
Aus logistischen Gründen ist die Verwendung von Konzentraten zur Herstellung der Imprägnierlösungen für die Feuchttücher vorteilhaft. Nachteilig ist, dass die Konzentrate beim Verdünnen vielfach die Tendenz zur Schaumbildung zeigen. Außerdem kann es zur Ausbildung von Gelphasen kommen, was zu einem erhöhten Zeitaufwand bei der Herstellung der Tranklösungen führt. In beiden Fällen wird der Durchsatz in der Produktion reduziert. Eine weitere Aufgabe der Erfindung hat somit darin bestanden, Tenside zur Verfügung zu stellen, mit deren Hilfe Konzentrate hergestellt werden können, die durch ihre Viskosität, Lagerstabilität, Schaumarmut beim Verdünnen und rasche Verdünnbarkeit eine technisch einfache und daher kostengünstige Herstellung der Feuchttücher gestattet.

### Beschreibung der Erfindung

Gegenstand der Erfindung sind Feuchttücher, die sich dadurch auszeichnen, dass sie mit linearen und/oder verzweigten Alkoholpolyglycolethern imprägniert sind.

Überraschenderweise wurde gefunden, dass nichtionische Tenside vom Typ der linearen und/oer verzweigten Alkoholpolyglycolether, vorzugsweise in Kombination mit Alkyloligoglucosiden die komplexe Aufgabe in ausgezeichneter Weise erfüllen. Imprägniermittel auf Basis von Alkoholpolyglycolethern erweisen sich in der Anmeldung als niedrigviskos und praktisch schaumfrei, in der Anwendung hinterlassen die damit getränkten Feuchttücher keine Streifen und beeinträchtigen den Glanz nicht. Konzentrate auf Basis der Alkoholpolyglycolether sind niedrigviskos und beim Verdünnen auf die Anwendungskonzentration besonders schaumarm.

### Alkoholpolyglycolether

Alkoholpolyglycolether stellen bekannte nichtionische Tenside dar, welche üblicherweise erhalten werden, indem man Ethylenoxid und/oder Propylenoxid, blockweise oder in random-Verteilung an geeignete primäre Alkohole oder Polyole addiert. Üblicherweise folgen die Polyglycolether der Formel (I), 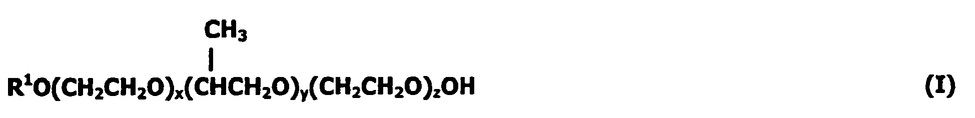 in der R¹ für einen linearen und/oder verzweigten Alkyl- und/oder Alkenylrest mit 6 bis 22, vorzugsweise 8 bis 18 und insbesondere 10 bis 12 Kohlenstoffatomen, einen Ethylenglycol-oder Glycerinrest, x und z unabhängig von einander für Zahlen von 1 bis 40 und y für Zahlen von 1 bis 40 steht; die Polyglycolether enthalten also zwingend mindestens eine Propylenoxid-Einheit. Typische Beispiele sind die Anlagerungsprodukte von durchschnittlich 1 bis 40, vorzugsweise 5 bis 30 und insbesondere 8 bis 15 Mol Ethylenoxid und/oder 1 bis 10, vorzugsweise 2 bis 5 Mol Propylenoxid an Fettalkohole, Oxoalkohole oder Alfole, wie etwa Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen sowie Ethylenglycol oder Glycerin. Die Einsatzmenge der Alkoholpolyglycolether kann bezogen auf die feuchten Tücher 0,05 bis 2 und vorzugsweise 0,1 bis 0,5 Gew.-% und bezogen auf die Konzentrate 10 bis 50, vorzugsweise 15 bis 25 Gew.-% betragen.

### Co-Tenside

In einer bevorzugten Ausführungsform der vorliegenden Erfindung werden die Mischether zusammen mit weiteren anionischen, nichtionischen, kationischen und/oder amphoteren bzw. zwitterionischen Tensiden eingesetzt.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside sind Alkylphenolpolyglycolether, Fettsäurepolyglycolester, Fettsäureamidpolyglycolether, Fettaminpolyglycolether, alkoxylierte Triglyceride, Mischether, Hydroxymischether, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Falbe (ed.), "Katalysatoren, Tenside und Mineralöladditive", Thieme Verlag, Stuttgart, 1978, S. 123-217 verwiesen.

Typische Beispiele für besonders geeignete Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Fettsäureglucamide, Alkylamidobetaine, Amphoacetale und/oder Proteinfettsäurekondensate, letztere vorzugsweise auf Basis von Weizenproteinen.

### Alkyl- und/oder Alkenyloligoglykoside

Anwendungstechnische Untersuchungen zeigen, dass Mischungen von Mischethern und Alkyl- und/oder Alkenyloligoglykosiden besonders vorteilhaft sind. Bei letzteren handelt es sich um bekannte nichtionische Tenside dar, die der Formel **(II)** folgen,

**R²O-[G]ₚ** (II)

in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften EP-A1 0301298 und WO 90/03977 verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R² kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Alkyl- und/oder Alkenyloligoglykoside können - bezogen auf die feuchten Tücher - in Mengen von 0,05 bis 2 und vorzugsweise 0,5 bis 1 Gew.-% und - bezogen auf die Konzentrate in Mengen von 10 bis 50, vorzugsweise 25 bis 25 Gew.-% eingesetzt werden, wobei das Gewichtsverhältnis Mischether zu Glykosid im Bereich von 10 : 90 bis 90 : 10, vorzugsweise 25 : 75 bis 75 : 25 und insbesondere 40 : 60 bis 60 : 40 liegen kann.

### Tissuepapiere und Tissuegewebe für Feuchtpapiere

Tissuepapiere, auf die sich die vorliegende Erfindung bezieht, können ein- oder mehrlagig aufgebaut sein. In der Regel weisen die Papiere ein Quadratmetergewicht von 10 bis 65, vorzugsweise 15 bis 30 g und eine Dichte von 0,6 g/cm³ und weniger auf. Beispiele für Tissuepapiere, auf sich die Erfindung erstrecken kann, sind neben Haushaltstüchern natürlich auch Toilettenpapiere, Papiertaschentücher, Gesichtsreinigungstücher, Abschminktücher, Erfrischungstücher und dergleichen. Neben den papierbasierten Tissues kommen auch entsprechende Tissuegewebe in Frage, die aus Faser- oder Fleecestoff hergestellt werden.

### Gewerbliche Anwendbarkeit

Ein letzter Gegenstand der Erfindung betrifft schließlich die Verwendung von linearen und/oder verzweigten Alkoholpolyglycolethern als Imprägniermittel zur Herstellung von Feuchttüchem, in denen sie in Mengen von 0,01 bis 2, vorzugsweise 0,5 bis 1 Gew.-% - bezogen auf die Tücher - eingesetzt werden können.

### Hilfs- und Zusatzstoffe

In einer weiteren Ausführungsform der Erfindung können die Feuchttücher weitere übliche Hilfs- und Zusatzstoffe, insbesondere Komplexbildner wie etwa Citronensäure, HEDP oder EDTA, welche sowohl zur Stabilisierung der Inhaltsstoffe, wie auch zur Verbesserung der Reinigungsleistung bei salzhaltigen Anschmutzungen (z.B. Wasserhärte) dienen, antibakterielle Wirkstoffe, wie etwa Wasserstoffperoxid oder Kationtenside, vorzugsweise Esterquats, sowie Hautpflegemittel enthalten. Als Hautpflegemittel kommen vor allem Rückfetter, Ölkomponenten und Emulgatoren in Frage, wie sie typischerweise im kosmetischen Produkten Anwendung finden.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhy-droxycarbonsäuren mit linearen oder verzweigten C₆-C₂₂-Fettalkoholen (vgl. DE 19756377 A1**),** insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Fnsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
> Anlagerungsprodukte von 2 bis 30 Mol Ethylenoxid und/ oder 0 bis 5 Mol Propylenoxid an an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
> Anlagerungsprodukte von 1 bis 15 Mol Ethylenoxid an Ricinusöl und/oder gehärtetes Ricinusöl;
> Anlagerungsprodukte von 15 bis 60 Mol Ethylenoxid an Ricinusöl und/oder gehärtetes Ricinusöl;
> Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
> Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
> Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
**>** Mono-, Di- und Trialkylphosphate sowie Mono-, Di- und/oder Tri-PEG-alkylphosphate und deren Salze;
> Wollwachsalkohole;
> Polysiloxan-Polyalkyl-Polyether-Copolymere bzw. entsprechende Derivate;
> Block-Copolymere z.B. Polyethylenglycol-30 Dipolyhydroxystearate;
> Polymeremulgatoren, z.B. Pemulen-Typen (TR-1,TR-2) von Goodrich;
> Polyalkylenglycole sowie
> Glycerincarbonat.

Die Anlagerungsprodukte von Ethylenoxid und/oder von Propylenoxid an Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Ctronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl-oder -Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglydne, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

Bei diesen Zubereitungen handelt es sich vorzugsweise um Emulsionen, vorzugsweise um Mikro- oder PIT-Emulsionen.

### Beispiele

**Beispiele 1 bis 4, Vergleichsbeispiel V1.** Verschiedene Imprägnierlösungen wurden durch einfaches Vermischen der Komponenten hergestellt; anschließend wurde das Schaumvermögen der Mischungen unter dynamischen Bedingungen nach der Frei-Fall-Kreislauf-Methode (1 Gew.-% Waschaktivsubstanz, 25°C, Förderrate 1 l/min) bestimmt. Zur Prüfung der Reinigungsleistung sowie der Glanzerhaltung wurden die Zubereitungen auf einen saugfähigen Träger (saugfähiges Tissuepapier, dreilagig, Gewicht 18 g/m2, 95 Gew.-% Recydingpapieranteil) aufgetragen. Zur Bestimmung des Reinigungsvermögens auf harten und elastischen Oberflächen wurde ein mit Testschmutz behandelter weißer Schmutzträger unter definierten Bedingungen mit den imprägnierten Tüchern gewischt. Der Reinigungseffekt wurde dabei photoelektrisch gegen den unbehandelten Schmutzträger (Standard = 100 %) gemessen. Zur Überprüfung der Glanzerhaltung wurde eine hochglänzende schwarze Kachel mit den imprägnierten Tüchern gereinigt und die Differenz mit Hilfe eines Glanzmeßgerätes (unbehandelter Standard = 100 %) bestimmt. Das Tauchnetzvermögen wurde schließlich gemäß DIN EN 1772 (0,1 Gew.-% Aktivsubstanz, 20 °C) gemessen. Die Zusammensetzung der Mischungen sowie die anwendungstechnischen Ergebnisse sind in Tabelle 1 zusammengefaßt. Die Beispiele 1 bis 4 sind erfindungsgemäß, Beispiel V1 dient zum Vergleich.

**Beispiele 5 und 6, Vergleichsbeispiel V2.** Verschiedene Imprägniermittelkonzentrate wurden hergestellt und ihre Viskosität (Höppler, 20 °C) sowie ihre Tendenz zur Schaumbildung und ihr äußeres Erscheinungsbild untersucht. Die Ergebnisse sind in Tabelle 2 zusammengefaßt. Die Beispiele 5 und 6 sind erfindungsgemäß, Beispiel V2 dient zum Vergleich.

**Tabelle 1**

| **Zusammensetzung der Imprägnierlösungen und anwendungstechnische Ergebnisse Mengenangaben als Gew.-%, Wasser ad 100 %** | | | | | |
|---|---|---|---|---|---|
| **Zusammensetzung** | **1** | **2** | **3** | **4** | **V1** |
| Träger | 25,0 | 25,0 | 25,0 | 25,0 | 25,0 |
| C₁₂-C₁₄-Kokosalkohol+5EO+4PO | 1,0 | 0,2 | 0,2 | - | - |
| Ethylenglycol+20EO+40PO | - | - | - | 0,2 | - |
| C₈-C₁₀ Alkyloligoglucosid | - | 0,8 | - | 0,8 | - |
| C₈-C₁₆ Alkyloligoglucosid | - | - | 0,8 | - | - |
| Isodecanol+8EO | - | - | - | - | 1,0 |
| Citronensäure | 0,1 | 0,1 | 0,1 | 0,1 | 0,1 |
| Isopropylalkohol | 5,0 | 5,0 | 5,0 | 5,0 | 5,0 |
| Wasserstoffperoxid | 0,8 | 0,8 | 0,8 | 0,8 | 0,8 |
| **Anwendungstechnische Eigenschaften** | | | | | |
| Schaumvermögen [ml] | 600 | 700 | 700 | 700 | 900 |
| Reinigungsvermögen [%-rel.] | 45 | 55 | 50 | 55 | 35 |
| Glanzerhaltung [%-rel.] | 75 | 95 | 85 | 95 | 70 |
| Netzvermögen [s] | 40 | 23 | 25 | 23 | 60 |

**Tabelle 2**

| **Zusammensetzung der Imprägnierlösungskonzentrate und anwendungstechnische Ergebnisse Mengenangaben als Gew.-%, Wasser ad 100 %** | | | |
|---|---|---|---|
| **Zusammensetzung** | **5** | **6** | **V2** |
| C₁₂-C₁₄-Kokosalkohol+5EO+4PO | 10,0 | 10,0 | - |
| C₈-C₁₀ Alkyloligoglucosid | 40,0 | - | - |
| C₈-C₁₆ Alkyloligoglucosid | - | 40,0 | - |
| Isodecanol+8EO | - | - | 50,0 |
| Bronidox ¹⁾ | 0,03 | 0,03 | 0,03 |
| Citronensäure | 0,1 | 0,1 | 0,1 |
| **Anwendungstechnische Eigenschaften** | | | |
| Viskosität [mPas] | 250 | 260 | > 3.000 |
| Erscheinungsbild | klar, homogen | klar, homogen | trüb |
| Tendenz zur Schaumbildung | gering | gering | stark |

| | | | |
|---|---|---|---|
| 1) Propylene Glycol (and) 5-Bromo-S-Nitro-1,3-Dioxane | | | |

## Patentansprüche

1. Feuchttücher, **dadurch gekennzeichnet, dass** sie mit linearen und/oder verzweigten Alkoholpolyglycolethern der Formel (I) enthalten, 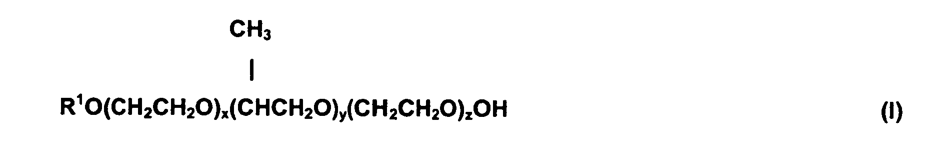 in der R¹ für einen Alkyl- und/oder Alkenylrest mit 6 bis 22 Kohlenstoffatomen, einen Ethylenglycol- oder Glycerinrest, x und z unabhängig von einander für Zahlen von 1 bis 40 und y für Zahlen von 1 bis 40 steht, imprägniert sind.

2. Feuchttücher nach dem Anspruch 1, **dadurch gekennzeichnet, dass** sie die Alkoholpolyglycolether in Mengen von 0,05 bis 2 Gew.-% - bezogen auf die feuchten Tücher - enthalten.

3. Feuchttücher nach mindestens einem der Ansprüche 1 bis 2, **dadurch gekennzeichnet, dass** sie weitere anionische, nichtionische, kationische und/oder amphotere bzw. zwitterionische Tenside enthalten.

4. Feuchttücher nach Anspruch 3, **dadurch gekennzeichnet, dass** sie Alkyl- und/oder Alkenyloligoglykoside enthalten.

5. Feuchttücher nach Anspruch 4, **dadurch gekennzeichnet, dass** sie Alkyl- und/oder Alkenyloligoglykoside der Formel (II) enthalten,
**R²O-[G]ₚ** (II)
in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

6. Feuchttücher nach mindestens einem der Ansprüche 4 bis 5, **dadurch gekennzeichnet, dass** sie die Alkyl- und/oder Alkenyloligoglykoside in Mengen von 0,05 bis 2 Gew.-% - bezogen auf die feuchten Tücher - enthalten.

7. Feuchttücher nach mindestens einem der Ansprüche 4 bis 6, **dadurch gekennzeichnet, dass** sie die Alkoholpolyglycolether und die Alkyl- und/oder Alkenyloligoglykoside im Gewichtsverhältnis 10 : 90 bis 90 : 10 enthalten.

8. Feuchttücher nach mindestens einem der Ansprüche 3 bis 7, **dadurch gekennzeichnet, dass** sie weitere übliche Hilfs- und Zusatzstoffe enthalten.

9. Verwendung von Mischungen aus linearen und/oder verzweigten Alkoholpolyglycolethern der Formel **(I)** enthalten, 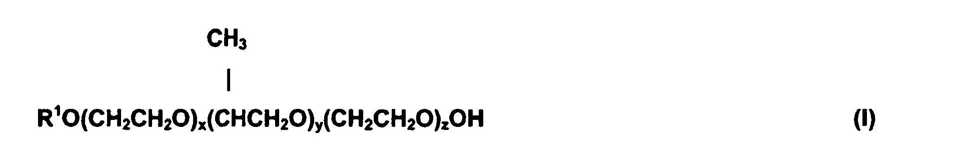 in der R¹ für einen Alkyl- und/oder Alkenylrest mit 6 bis 22 Kohlenstoffatomen, einen Ethylenglycol- oder Glycerinrest, x und z unabhängig von einander für Zahlen von 1 bis 40 und y für Zahlen von 1 bis 40 steht, als Imprägniermittel zur Herstellung von Feuchttüchern.

## Claims

1. Wet wipes, **characterized in that** they are impregnated with linear and/or branched alcohol polyglycol ethers corresponding to formula (I): 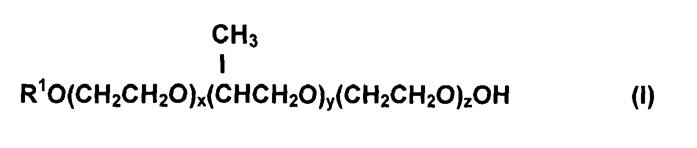 in which R¹ is an alkyl and/or alkenyl group containing 6 to 22 carbon atoms, an ethylene glycol or glycerol residue, x and z independently of one another are numbers of 1 to 40 and y is a number of 1 to 40.

2. Wet wipes as claimed in claim 1, **characterized in that** they contain the alcohol polyglycol ethers in quantities of 0.05 to 2% by weight, based on the wet wipes.

3. Wet wipes as claimed in at least one of claims 1 to 2, **characterized in that** they contain other anionic, nonionic, cationic and/or amphoteric or zwitterionic surfactants.

4. Wet wipes as claimed in claim 3, **characterized in that** they contain alkyl and/or alkenyl oligoglycosides.

5. Wet wipes as claimed in claim 4, **characterized in that** they contain alkyl and/or alkenyl oligoglycosides corresponding to formula (II):
**R²O-[G]ₚ** (II)
in which R² is an alkyl and/or alkenyl group containing 4 to 22 carbon atoms, G is a sugar unit containing 5 or 6 carbon atoms and p is a number of 1 to 10.

6. Wet wipes as claimed in at least one of claims 4 to 5, **characterized in that** they contain the alkyl and/or alkenyl oligoglycosides in quantities of 0.05 to 2% by weight, based on the wet wipes.

7. Wet wipes as claimed in at least one of claims 4 to 6, **characterized in that** they contain the alcohol polyglycol ethers and the alkyl and/or alkenyl oligoglycosides in a ratio by weight of 10:90 to 90:10.

8. Wet wipes as claimed in at least one of claims 3 to 7, **characterized in that** they contain other typical auxiliaries and additives.

9. Use of mixtures of linear and/or branched alcohol polyglycol ethers corresponding to formula **(I):** 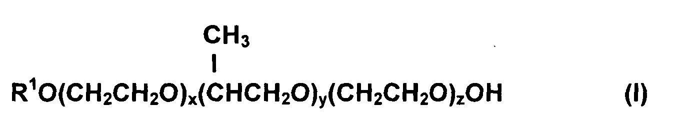 in which R¹ is an alkyl and/or alkenyl group containing 6 to 22 carbon atoms, an ethylene glycol or glycerol residue, x and z independently of one another are numbers of 1 to 40 and y is a number of 1 to 40, as impregnating agents for the production of wet wipes.

## Revendications

1. °) Lingettes
**caractérisées en ce qu'**
elles sont imprégnées de polyglygol éthers d'alcool linéaires et/ou ramifiés de formule (I) 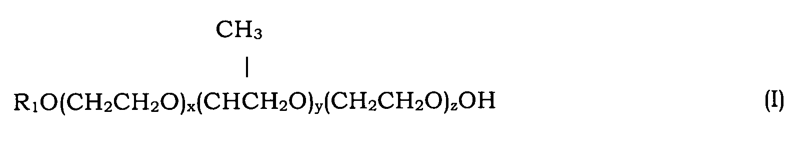 dans laquelle R¹ représente un radical alkyle et/ou alcényle ayant de 6 à 22 atomes de carbone, un radical éthylène glycol ou glycérol, x et z représentent indépendamment l'un ou l'autre des nombres de 1 à 10 et y représente des nombres de 1 à 40.

2. °) Lingettes selon la revendication 1,
**caractérisées en ce qu'**
elles renferment les polyglycol éthers d'alcool en quantité de 0,05 % à 2 % en poids - par rapport aux lingettes -.

3. °) Lingettes selon au moins l'une des revendications 1 et 2,
**caractérisées en ce qu'**
elles renferment d'autres tensioactifs anoniques, non ioniques, cationiques et/ou amphotères ou zwitterioniques.

4. °) Lingettes selon la revendication 3,
**caractérisées en ce qu'**
elles renferment des alkyl- et/ou alcényl oligo glycosides.

5. °) Lingettes selon la revendication 3,
**caractérisées en ce qu'**
elles renferment des alkyl- et/ou alcényl oligo glycosides de formule (II)
R₂O-[G]ₚ (II)
dans laquelle R² représente un radical alkyle et/ou alcényle ayant de 4 à 22 atomes de carbone, G un radical sucre ayant 5 ou 6 atomes de carbone et p représente des nombres de 1 à 10.

6. °) Lingettes selon au moins l'une des revendications 4 et 5,
**caractérisées en ce qu'**
elles renferment les alkyls- et/ou alcényls oligo glycosides en quantité de 0,05 % à 2 % en poids - par rapport aux lingettes -.

7. °) Lingettes selon au moins l'une des revendications 4 à 6,
**caractérisées en ce qu'**
elles renferment les polyglycol éthers d'alcool et les alkyl- et/ou alcényl oligo glycosides dans un rapport pondéral de 10/90 à 90/10.

8. °) Lingettes selon au moins l'une des revendications 3 à 7,
**caractérisées en ce qu'**
elles renferment d'autres adjuvents et additifs usuels.

9. °) Utilisation de mélanges de polyglycol éthers d'alcool linéaires et/ou ramifiés de formule (I) 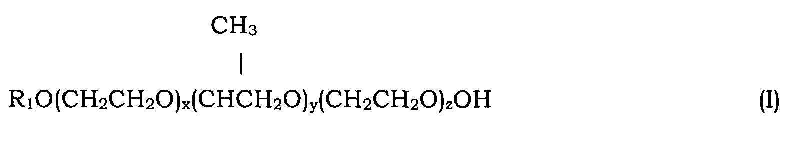 dans laquelle R1 représente un radical alkyle et/ou alcényle ayant de 6 à 22 atomes de carbone, un radical éthylène glycol ou glycérol, x et z représentent indépendamment l'un de l'autre des nombres de 1 à 40 et y représente des nombres de 1 à 40 en tant qu'agent d'imprégnation pour la fabrication de lingettes.
